# EUROPÄISCHE PATENTSCHRIFT

(11) **EP 2 208 064 B1**
(45) Veröffentlichungstag und Bekanntmachung des Hinweises auf die Patenterteilung: **20.07.2011**
(21) Anmeldenummer: 08848834.1
(22) Anmeldetag: 12.11.2008
(51) Int. Cl.: G01N 33/483, G01N 33/487, G01N 33/49, G01N 33/493, G01N 33/50, A61B 5/00, A61B 5/15

(54) **AUSTAUSCHBARE VERBRAUCHSMITTEL-KASSETTE MIT INTEGRIERTEM LUFTFILTER FÜR ANALYSEGERÄTE**
REPLACEABLE CARTRIDGE FOR CONSUMABLES HAVING INTEGRATED AIR FILTER FOR ANALYSIS DEVICES
CASSETTE CONSOMMABLE REMPLAÇABLE AVEC FILTRE A AIR INTEGRE POUR APPAREILS D'ANALYSE

(30) Priorität: 13.11.2007 US 987450 P
(43) Veröffentlichungstag der Anmeldung: 21.07.2010
(73) Patentinhaber: F. Hoffmann-La Roche AG, 4070 Basel (CH); Roche Diagnostics GmbH, 68305 Mannheim (DE)
(72) Erfinder: SPRENGERS, Wolfgang, A-8081 Heiligen Kreuz Am Waasen (AT); EBNER, Berndt, A-8010 Graz (AT); RIEGELNEGG, Andreas Johann, A-8044 Graz-Weinitzen (AT)
(74) Vertreter: Babeluk, Michael
(86) Internationale Anmeldenummer: PCT/EP2008/065334
(87) Internationale Veröffentlichungsnummer: WO 2009/062938

(56) Entgegenhaltungen:
- WO-A-2006/132666
- US-A1- 2004 189 311
- US-A1- 2006 013 726

## Beschreibung

Die Erfindung betrifft einen Analysator, vorzugsweise für die Analyse von Körperflüssigkeiten, welcher eine oder mehrere austauschbare Kassetten (Consumables) aufweist, die Betriebsfluide, Betriebsmittel und/oder Verbrauchsmittel enthalten und in entsprechende Aufnahmen des Analysators einsetzbar sind, wobei der Analysator ein System zum Austausch von Umgebungsluft aufweist, welches eingangsseitig des Analysators eine Filtereinheit aufweist. Weiterhin betrifft die Erfindung Kassetten, welche austauschbar in einen derartigen Analysator einsetzbar ist und welche Betriebsfluide, Betriebsmittel und/oder Verbrauchsmittel zum Betrieb des Analysators enthalten

Als austauschbare Consumables werden turnusmäßig (beispielsweise nach Ablauf einer bestimmten Zeit oder nach Durchführung einer bestimmten Anzahl von Messungen oder Verbrauch einer bestimmten Menge des Betriebsmittels) austauschbare Verbrauchsmaterialien bzw. Behälter oder Packs bezeichnet, welche Verbrauchsmaterialien enthalten, die vom Bedienungspersonal in einen Analysator, insbesondere in einen Analysator zur Analyse von Körperflüssigkeiten, eingesetzt werden können.

Solche Analysatoren zur Analyse von Körperflüssigkeiten werden beispielsweise als portable Analysatoren zur Bestimmung von POC (Point Of Care) Parametern, nämlich der Blutgase (O₂, CO₂, pH), der Elektrolyte (z.B. K⁺, Na⁺, Ca⁺⁺, Cl⁻) der Metabolite (z.B. Glukose und Laktat), des Hämatokrits, der Hämoglobinparameter (z.B. tHb, SO₂, etc.) und Bilirubin entwickelt und werden vor allem zur dezentralen Bestimmung der oben genannten Parameter in Vollblutproben eingesetzt. Anwendungen in der Veterinärmedizin und die Verwendung von Serum-, Plasma-, Harn- und Dialysat-Proben sind möglich.

Idealerweise sollen solche Analysatoren auch für den "untrainierten" Benutzer, einfach und intuitiv zu bedienen sein. Ein weiteres vorteilhaftes Merkmal ist es, wenn das Gerät aus Sicht des Anwenders "virtually maintenance free" zu betreiben ist. Unter "virtually maintenance free" wird allgemein ein möglichst wartungsfreies System verstanden, bei dem auch der (technisch) ungeschulte Anwender - ähnlich wie z.B. bei einem Tintenstrahldrucker - zum laufenden Betrieb lediglich in Form von Kassetten und/oder Modulen vorliegende Verbrauchsmaterialien tauschen kann. Alle Verbrauchsmaterialien sollen durch einfache intuitive Handgriffe vom Anwender tauschbar sein.

In einem möglichen Konzept eines solchen Analysators können so beispielsweise Verbrauchsmaterialien in Consumables wie folgt zusammengefasst sein:
Consumable1 :
   Sensorkassette, welche alle oder zumindest einen Teil der für die Analytbestimmung benötigten Sensoren enthält.
Consumable2 :
   Flüssigkeitsbehälter bzw. Fluidpack, welches die zum Betrieb des Analysators benötigten Funktionsfluide (z. B. Kalibrationslösungen, Waschlösungen, Referenzflüssigkeiten, bestimmte zum Betrieb benötigte Reagenzienlösungen etc.) enthält.
   Optional können hier auch weitere Elemente oder Funktionalitäten wie das gesamte Fluidiksystem oder Teile dessen, die Probeneingabevorrichtung oder auch weitere sensorische Komponenten enthalten sein.
Consumable3 :
   Druckerpapier für einen internen Drucker.
Consumable4:
   Optional können weitere Consumables angeboten werden, beispielsweise eine Kassette mit ampullierten Referenzlösungen zur Durchführung einer automatisierten Qualitätskontrolle (aQC), welche durch einfache intuitive Handgriffe vom Anwender selbst tauschbar sein soll.

Die hier geschilderte Untergliederung der Consumables soll nur als beispielhafte Ausführungsvariante gedacht sein. Es ist auch denkbar, dass (Teil-)Funktionalitäten oder (Teil-)Elemente mehrerer Consumables zusammengefasst werden, so dass beispielsweise weniger oder sogar nur ein Consumable benötigt wird. Andererseits ist es auch denkbar, dass (Teil-)Funktionalitäten oder (Teil-)Elemente einzelner Consumables auf mehrere verteilt werden.

Die Consumables werden untereinander bzw. mit dem Analysator durch aufeinander abgestimmte Schnittstellen, z.B. in Form fluidischer Andocknippel, verbunden. Das mechanische Verbinden der Consumables mit den respektiven Gegen parts kann durch einen einfachen manuellen Bewegungsablauf direkt durch den Benutzer erfolgen, oder durch im Gerät befindliche Antriebe, die die Ankopplung automatisch durchführen, nachdem der Benutzer die Kassette nur in "Position" bzw. in eine entsprechende Aufnahme gebracht hat.

Medizinische Analysatoren besitzen viele elektronische Bauelemente, welche zu einem zuverlässigen Betrieb eine möglichst konstante Umgebungstemperatur brauchen.

Des Weiteren müssen auch die sensorischen Bereiche des Analysators oft auf bestimmte Temperaturbereiche temperiert werden, um den Sensoren eine möglichst ideale Betriebsumgebung zu bieten. So sollten beispielsweise Enzymsensoren zur Glukose- oder Laktatbestimmung aus Haltbarkeitsgründen auf 30°C temperiert werden, während beispielsweise Sensoren zur Blutgasbestimmung auch bevorzugt bei 37°C betrieben werden können.

Auch die beispielsweise im Fluidpack enthaltenen Funktionsfluide weisen bestimmte bevorzugte Temperaturbedingungen auf. So können beispielsweise durch reduzierte Lagerungstemperaturen bestimmte Lösungen länger haltbar sein.

Weiterhin werden medizinische Analysatoren weltweit unter unterschiedlichsten klimatischen Randbedingungen eingesetzt, wodurch diese in einem großen Temperatur- und/oder Luftfeuchte-Bereich zuverlässig funktionieren müssen.

Zusammengefasst zeigt sich, dass medizinische Analysatoren zuverlässig über ein gutes Temperaturmanagement verfügen müssen und hierzu Vorrichtungen aufweisen müssen, welche möglichst unabhängig von Umgebungsbedingungen und momentanem Betriebszustand des Analysators eine geregelte Betriebstemperatur ermöglichen.

Ein Bestandteil des Temperaturmanagements solcher medizinischer Analysatoren sind Lüftungsysteme, welche gezielt Abwärme abführen können, indem sie über bestimmte Lüftungskanäle z.B. Umgebungsluft zur Kühlung ansaugen.

Da mit der Umgebungsluft auch darin enthaltene Fremdkörper (z.B. Staub, Partikel, Feuchtigkeitströpfchen) angesaugt werden und somit ins Innere des Analysators gelangen können, weisen solche Lüftungssysteme insbesondere in ihren Ansaugkanälen Luftfilter auf, welche die eingesaugte Luft filtern, um das Innere des Analysators möglichst vor solchen Verunreinigungen zu schützen.

Als Luftfilter werden allgemein Abscheider bezeichnet, die unerwünschte Schwebstoffe wie Keime, Pollen, Stäube oder Gase aus der Luft herausfiltern. Es handelt sich dabei um filternde Abscheider, die in einem Filtermedium Substanzen aus einem Luftstrom entfernen. Als Filtermedium (Kollektoren) kommen meist Fasern oder Körner zum Einsatz. Es wird bspw. in Faserschichtfilter, Schüttschichtfilter und Filter mit festem Medium (seltener, wie Sinterschichten, Keramik) unterschieden.

Durch das Durchströmen der Luftfilter mit Umgebungsluft lagern sich Verunreinigungen auf den inneren Oberflächen des Luftfilters an, wodurch dessen Filterleistung im Lauf des Einsatzes abnimmt.

Um möglichst immer eine gute Filterleistung, insbesondere einen hohen Luftdurchsatz bei gleichzeitigem Rückhalt möglichst aller Schwebstoffe, zu gewährleisten, müssen daher Luftfilter in regelmäßigen Abständen ausgetauscht werden.

Solche Austauschschritte müssen bei bekannten Geräten durch den Nutzer durchgeführt werden, welcher den verschmutzen Luftfilter manuell aus dem Gerät entfernen und durch einen neuen Luftfilter ersetzen muss.

Da solche Austauschprozesse nicht zum täglichen Routinebetrieb eines medizinischen Analysators gehören, werden diese nicht vorrangig geschult und geübt, so dass diese daher fehleranfälliger sind oder überhaupt unterlassen werden.

Weiterhin werden oft die Einsatzdauern solcher Luftfilter in einem Gerät oder auch deren Verschmutzungsgrad nicht überwacht, so dass der Benutzer nicht auf einen fälligen Luftfilterwechsel hingewiesen wird.

Luftfilter sind bei den im Stand der Technik bekannten Analysatoren als zusätzliche Consumables ausgeführt, welche in den Analysator eingesetzt werden und für welche eine eigene Handhabungsschulung und Lagerhaltung notwendig sind. Dadurch können Handhabungsprobleme (z.B. falsches Einsetzen) oder auch Verfügbarkeitsprobleme (fehlende Lagerbestände) auftreten.

Folge solcher Probleme können beispielsweise Defekte am Analysator durch eingedrungene Verunreinigungen oder die zumindest zeitweise Sperrung des Analysators für weitere Messungen aufgrund einer falschen Temperierung sein.

Ziel der vorliegenden Erfindung ist es, die oben genannten Nachteile in einem Analysator mit austauschbaren Kassetten zu vermeiden.

Erfindungsgemäß werden die aufgezeigten Probleme dadurch gelöst, dass die Filtereinheit in zumindest eine der auswechselbaren Kassetten integriert ist. Der Luftfilter wird somit nicht mehr als separates Ersatzteil ausgeführt, sondern in ein bestehendes Consumable eingebaut.

Hierdurch wird sichergestellt, dass die Filtereinheit automatisch in bestimmten Abständen turnusmäßig mit dem Consumable ausgetauscht wird.

In einer bevorzugten Ausführungsform weist die austauschbare Kassette mit der integrierten Filtereinheit einen Lüftungskanal auf, der saug- oder druckseitig mit einer im Analysator angeordneten Luftfördereinrichtung, vorzugsweise einem Ventilator und/oder damit in Verbindung stehenden analysatorseitigen Luftführungskanälen, verbunden ist.

Bevorzugt kann die Filtereinheit in einem Fluidpack integriert sein und zusammen mit diesem getauscht werden.

Durch den turnusmäßigen Wechsel des Fluidpacks, beispielsweise nach einer vorbestimmten Zeit oder nach Verbrauch der darin enthaltenen Betriebsmittel, wird somit die Filtereinheit automatisch mit ausgetauscht, ohne dass zusätzliche manuelle Schritte seitens des Nutzers notwendig werden. Hierdurch wird auch keine separate Schulung zum Tausch des Luftfilters mehr benötigt und Fehler durch falsche Durchführung des Filterwechsels werden ausgeschlossen. Weiterhin kann durch eine angemessen hohe Dimensionierung der Filterleistung und Filterkapazität erreicht werden, dass ein nennenswertes Nachlassen der Filterleistung innerhalb der turnusgemäßen Einsatzzeit des Consumables vermieden werden kann. Hierdurch kann auf zusätzliche Messvorrichtungen im Analysator zur Bestimmung des Verschmutzungsgrades des Luftfilters verzichtet werden. Zusätzlich sind durch die Integration in bereits existierende Consumables keine separate Lagerhaltung und kein separater Vertrieb von Luftfiltern mehr notwendig.

In einer besonders vorteilhaften Ausführungsvariante der Erfindung weist der Lüftungskanal in der austauschbaren Kassette eine bevorzugt zum Bodenbereich des Analysators gerichtete Ausgangsöffnung auf, in deren Bereich die Filtereinheit angeordnet ist. Dadurch weist die Filtereinrichtung bei eingesetzter Kassette eine horizontal nach oben ausgerichtete Anströmfläche auf, auf der sich das Filtrat (Staub, Partikel, etc.) ablagert und beim Kassettenwechsel nicht in den Analysator gelangen kann.

Weiters kann der Lüftungskanal in der austauschbaren Kassette beispielsweise U-förmig geführt sein und eingangsseitig mit einem Ansaugkanal, der bevorzugt im Bodenbereich des Analysators angeordnet ist, in Verbindung stehen.

Die Erfindung wird im Folgenden anhand von Zeichnungen näher erläutert. Es zeigen:
- Fig. 1: einen erfindungsgemäßen Analysator mit austauschbarer Kassette in einer schematischen Darstellung;
- Fig. 2: eine Variante des Analysators gemäß Fig. 1; sowie
- Fig. 3: eine weitere Ausführungsvariante des erfindungsgemäßen Analysa- tors mit mehreren austauschbaren Kassetten.

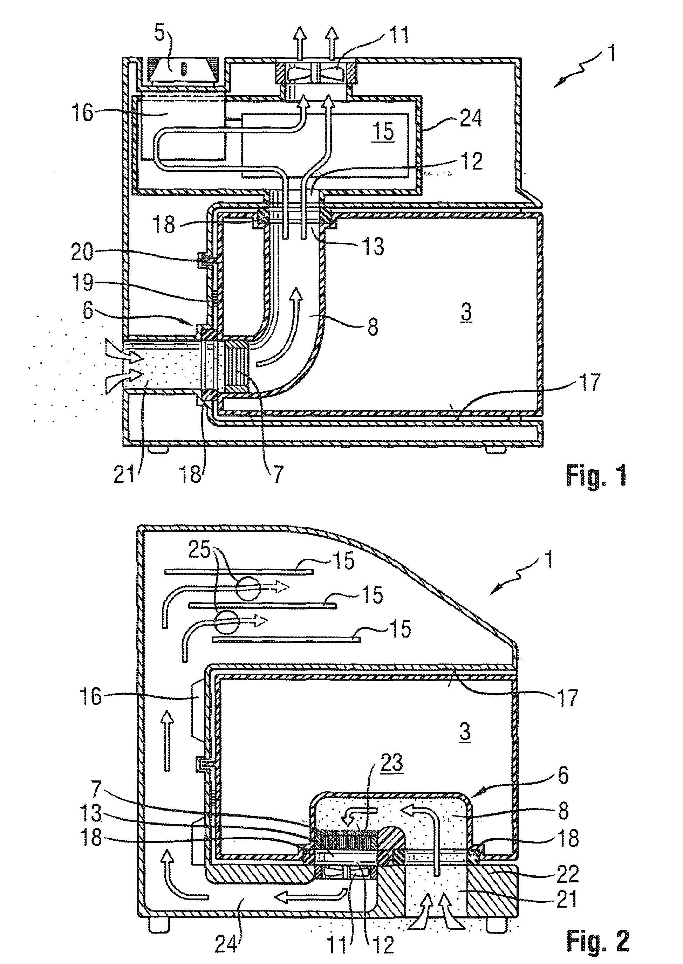

Der in Fig. 1 dargestellte Analysator 1 wird beispielsweise für medizinische Analysen eingesetzt und weist eine austauschbare Kassette 3 auf, die in eine entsprechende Aufnahme 17 des Analysators 1 einsetzbar ist. Bei der Kassette 3 handelt es sich beispielsweise um ein Fluidpack, in welchem die für den Betrieb des Analysators benötigten Betriebs- und Verbrauchsmittel aufgenommen sind. Der Analysator weist ein System 6 zum Austausch von Umgebungsluft auf, welche im Wesentlichen eine Filtereinheit 7 und einen saugseitig im Analysator angeordneten Ventilator 11 sowie die nötigen Luftführungskanäle aufweist. Erfindungsgemäß ist die Filtereinheit 7 in der auswechselbaren Kassette 3 integriert und wird mit dieser turnusmäßig ausgewechselt.

Die die Filtereinrichtung 7 aufnehmende austauschbare Kassette 3 weist einen Luftführungskanal 8 mit einer Ausgangsöffnung 13 auf, die mit einer entsprechenden Eintrittsöffnung 12 eines Luftführungskanals 24 im Analysator 1 in Verbindung steht und zu dieser - nach dem Einsetzen der Kassette 3 in die Aufnahme 17 - beispielsweise mit Hilfe eines Dichtringes 18, abgedichtet ist. In gleicher Weise erfolgt die Abdichtung des Luftführungskanals 8 im Bereich der Filtereinheit 7 zum Ansaugkanal 21 des Analysators mit einem Dichtelement 18.

Nach Einsetzen der Kassette durch den Benutzer rasten automatisch fluidische Andocknippel 19 sowie elektrische Anschlüsse 20 ein und stellen die für den Betrieb notwendigen Verbindungen her. Der Luftführungskanals 24 des Analysators ist auf die zu kühlenden Komponenten, beispielsweise elektronische Auswerteeinheit 15, Netzteil 16, ggf. Thermostatisiereinrichtung etc., gerichtet.

Mit 5 ist eine austauschbare Kassette für Druckerpapier für einen internen Drucker bezeichnet.

In der Ausführungsvariante gemäß Fig. 2 weist der Lüftungskanal 8 in der austauschbaren Kassette 3 eine zum Bodenbereich 22 des Analysators gerichtete Ausgangsöffnung 13 auf, in deren Bereich die Filtereinheit 7 angeordnet ist. Diese weist eine horizontal ausgerichtete Anströmfläche 23 auf, auf welcher sich die aus der Umgebungsluft gefilterten Schwebeteilchen in Form einer strichliert angedeuteten Staubschicht ablagern. Beim Kassettenwechsel verbleibt die Staubschicht in der Filtereinheit 7 und kann nicht in den Analysator gelangen.

Der Lüftungskanal 8 ist in der Variante gemäß Fig. 2 beispielsweise U-förmig geführt und steht eingangsseitig mit einem Ansaugkanal 21 im Bodenbereich 22 des Analysators in Verbindung.

Die Filtereinheit 7 kann derartig in das Fluidpack 3, beispielsweise in dessen Sockel, integriert sein, dass durch die korrekte Lage des Fluidpacks im Analysator automatisch die Öffnungen im Fluidpack mit den korrespondierenden Öffnungen 12, 21 im Analysator 1 verbunden werden, um den entsprechenden Luftstrom im Analysator zu bewirken. Dies wird durch entsprechende Führungen und die Dichtungen 18 am Fluidpack 3 oder im Analysator 1 verwirklicht. Der Luftstrom kann den Analysator beispielsweise durch seitliche Austrittsöffnungen 25 im Bereich der Auswerteeinheit 15 verlassen.

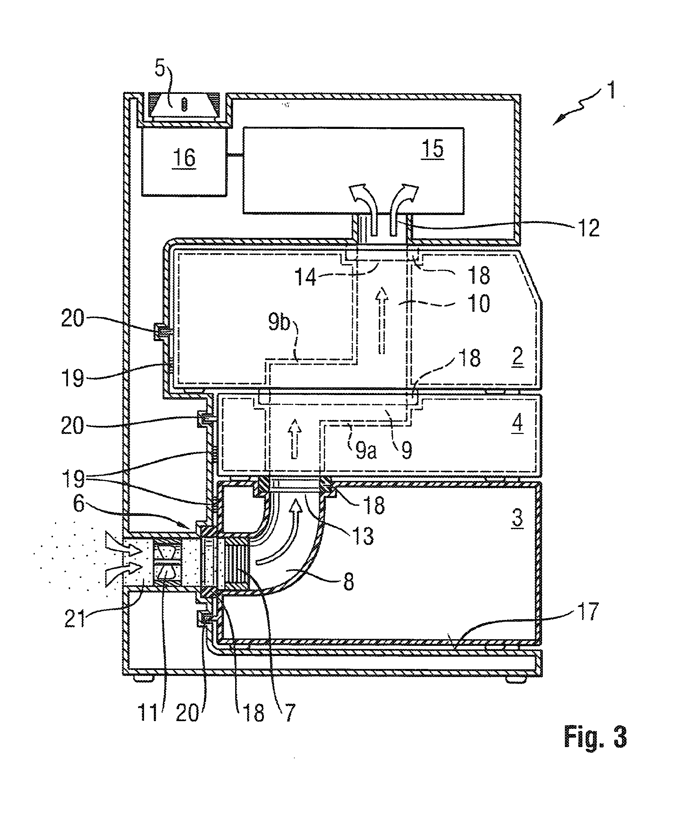

In der Ausführungsvariante gemäß Fig. 3 ist ein Analysator 1 mit einer austauschbaren Sensorkassette 2, einem austauschbaren Fluidpack 3 und einer austauschbaren Kassette 4 für Qualitätskontrollmedien dargestellt, wobei die Filtereinheit 7 auch hier im Fluidpack 3 integriert ist. Der Luftführungskanal 8 ist hier druckseitig mit einer im Ansaugkanal 21 des Analysators 1 angeordneten Luftfördereinrichtung (Ventilator 11) verbunden.

Die Reagenzienkassette 3 kann auch Luftausgänge aufweisen, die mit entsprechenden Öffnungen in einem anderen Disposable in Verbindung stehen, so dass auch diese mit gefilterter Luft beschickt werden können. Wie strichliert angedeutet, können Teile der Luftführungskanäle 9 und 10 durch entsprechende Formgebung von Wandbereichen 9a, 9b benachbarter austauschbarer Kassetten 2, 4 geformt sein, wobei die Ausgangsöffnung 14 der Kassette 2 mit der Öffnung 12 im Analysator kommuniziert.

Die Filtereinheit 7 kann als Filtermaterial ein Faserschichtfilter, ein Schüttschichtfilter oder ein Festkörperfilter aufweisen. Die Filtereinheit kann auch aus mehreren einzelnen Filterlagen bestehen.

Das Luftfilter muss nicht notwendigerweise Bestandteil des Fluidpacks 3 sein, sondern kann auch in ein anderes Disposable 2, 4, 5 integriert sein.

## Patentansprüche

1. Analysator (1), vorzugsweise für die Analyse von Körperflüssigkeiten, welcher eine oder mehrere austauschbare Kassetten (2, 3, 4, 5) aufweist, die - Betriebsfluide, Betriebsmittel und/oder Verbrauchsmittel enthalten und in entsprechende Aufnahmen (17) des Analysators (1) einsetzbar sind, wobei der Analysator (1) ein System (6) zum Austausch von Umgebungsluft für das Temperaturmanagement des Analysators aufweist, welches eingangsseitig des Analysators (1) eine Filtereinheit (7) zum Filtern der auszutauschenden Umgebungsluft aufweist, **dadurch gekennzeichnet, dass** die Filtereinheit (7) in zumindest eine der auswechselbaren Kassetten (2, 3, 4, 5) integriert ist.

2. Analysator (1) nach Anspruch 1, **dadurch gekennzeichnet, dass** die austauschbare Kassette (3) mit der integrierten Filtereinheit (7) einen Lüftungskanal (8) aufweist, der saug- oder druckseitig mit einer im Analysator (1) angeordneten Luftfördereinrichtung, vorzugsweise einem Ventilator (11), verbunden ist.

3. Analysator (1) nach Anspruch 2, **dadurch gekennzeichnet, dass** der Lüftungskanal (8) in der austauschbaren Kassette (3) eine bevorzugt zum Bodenbereich (22) des Analysators gerichtete Ausgangsöffnung (13) aufweist, in deren Bereich die Filtereinheit (7) angeordnet ist, welche eine horizontal ausgerichtete Anströmfläche (23) aufweist.

4. Analysator (1) nach Anspruch 3, **dadurch gekennzeichnet, dass** der Lüftungskanal (8) in der austauschbaren Kassette (3) beispielsweise U-förmig geführt ist und eingangsseitig mit einem Ansaugkanal (21), der bevorzugt im Bodenbereich (22) des Analysators angeordnet ist, in Verbindung steht.

5. Analysator (1) nach einem der Ansprüche 2 oder 4, **dadurch gekennzeichnet, dass** der Luftführungskanal (8) nach dem Einsetzen der austauschbaren Kassette (3) in den Analysator mit einer entsprechenden Eintrittsöffnung (12) eines Luftführungskanals (24) im Analysator (1) in Verbindung steht.

6. Analysator (1) nach Anspruch 5, **dadurch gekennzeichnet, dass** der Luftführungskanal (24) des Analysators auf zu kühlende Komponenten (15, 16) des Analysators (1) gerichtet ist.

7. Analysator (1) nach einem der Ansprüche 1. bis 6, **dadurch gekennzeichnet, dass** der Analysator (1) zumindest eine austauschbare Sensorkassette (2), zumindest ein austauschbares Fluidpack (3) und zumindest eine austauschbare Kassette (4) mit Qualitätskontrollmedien aufweist, wobei die Filtereinheit (7) bevorzugt im Fluidpack (3) integriert ist.

8. Analysator (1) nach einem der Ansprüche 1 bis 7, **dadurch gekennzeichnet, dass** die Filtereinheit (7) ein Faserschichtfilter, ein Schüttschichtfilter oder ein Festkörperfilter aufweist.

9. Kassette (2, 3, 4, 5) welche austauschbar in einen Analysator (1), vorzugsweise in einen Analysator zur Analyse von Körperflüssigkeiten einsetzbar ist, der ein System (6) zum Austausch von Umgebungsluft für das Temperaturmanagement des Analysators aufweist, und welche Betriebsfluide, Betriebsmittel und/oder Verbrauchsmittel zum Betrieb des Analysators enthält, **dadurch gekennzeichnet, dass** eine Filtereinheit (7) zum Filtern der auszutauschenden Umgebungsluft in die austauschbare Kassette (2, 3, 4, 5) integriert ist.

10. Kassette nach Anspruch 9, **dadurch gekennzeichnet, dass** die Filtereinheit (7) ein Faserschichtfilter, ein Schüttschichtfilter oder ein Festkörperfilter aufweist.

11. Kassette nach Anspruch 9, **dadurch gekennzeichnet, dass** die Kassette ein Fluidpack (3) ist, welches zum Betrieb des Analysators benötigten Funktionsfluide, insbesondere Kalibrationslösungen, Waschlösungen, Referenzflüssigkeiten oder bestimmte zum Betrieb benötigte Reagenzienlösungen, enthält.

12. Kassette nach Anspruch 9, **dadurch gekennzeichnet, dass** die Kassette einen Lüftungskanal (8) aufweist, der saug- oder druckseitig mit einer im Analysator (1) angeordneten Luftfördereinrichtung, vorzugsweise einem Ventilator (11) und/oder damit in Verbindung stehenden analysatorseitigen Luftführungskanälen (21, 24), verbindbar ist.

## Claims

1. Analyzer (1), preferably for analysing body fluids, which has one or more exchangeable cassettes (2, 3, 4, 5) that contain operating fluids, operating materials and/or consumables and is insertable into corresponding holders (17) of the analyzer (1), wherein the analyzer (1) has a system (6) for exchanging ambient air for the temperature management of the analyzer, which has a filter unit (7) on the inlet side of the analyzer (1) to filter the ambient air that needs to be exchanged, **characterised in that** the filter unit (7) is integrated into at least one of the exchangeable cassettes (2, 3, 4, 5).

2. Analyzer (1) according to claim 1, **characterised in that** the exchangeable cassette (3) with the integrated filter unit (7) has a ventilation channel (8) which on the suction or pressure side is connected to an air-conveying device located in the analyzer (1) and preferably to a ventilator (11).

3. Analyzer (1) according to claim 2, **characterised in that** the ventilation channel (8) in the exchangeable cassette (3) has an outlet opening (13) that is preferably directed towards the bottom area (22) of the analyzer, in which area the filter unit (7) is arranged which has a horizontally aligned approach-flow surface (23).

4. Analyzer (1) according to claim 3, **characterised in that** the ventilation channel (8) in the exchangeable cassette (3) is for example U-shaped and on the inlet side communicates with a suction channel (21) which is preferably located in the bottom area (22) of the analyzer.

5. Analyzer (1) according to one of the claims 2 or 4, **characterised in that** after the exchangeable cassette (3) has been inserted into the analyzer, the ventilation channel (8) communicates with a corresponding inlet opening (12) of an air-guiding channel (24) in the analyzer (1).

6. Analyzer (1) according to claim 5, **characterised in that** the air-guiding channel (24) of the analyzer is directed towards the components (15, 16) of the analyzer (1) that need to be cooled.

7. Analyzer (1) according to one of the claims 1 to 6, **characterised in that** the analyzer (1) has at least one exchangeable sensor cassette (2), at least one exchangeable fluid pack (3) and at least one exchangeable cassette (4) containing quality control media, wherein the filter unit (7) is preferably integrated into the fluid pack (3).

8. Analyzer (1) according to one of the claims 1 to 7, **characterised in that** the filter unit (7) comprises a fibre layer filter, a granular bed filter or a solid body filter.

9. Cassette (2, 3, 4, 5) which is exchangeable insertable into an analyzer (1) and preferably into an analyzer for analysing body fluids which, comprises a system (6) for exchanging ambient air for the temperature management of the analyzer, and which contains operating liquids, operating materials and/or consumables for operating the analyzer (1), **characterised in that** a filter unit (7) for filtering ambient air that is to be exchanged is integrated into the exchangeable cassette (2, 3, 4, 5).

10. Cassette according to claim 9, **characterised in that** the filter unit (7) comprises a fibre layer filter, a granular bed filter or a solid body filter.

11. Cassette according to claim 9, **characterised in that** the cassette is a fluid pack (3) which contains which contains the functional fluids required to operate the analyzer and in particular calibration solutions, washing solutions, reference liquids or certain reagent solutions required for the operation.

12. Cassette according to claim, **characterised in that** the cassette has a ventilation channel (8) which can be connected to an air-conveying device in the analyzer on the suction or pressure side and preferably to a ventilator (11) and/or to air-guiding channels (21, 24) that are connected thereto on the analyzer side.

## Revendications

1. Analyseur (1), prévu de préférence pour l'analyse de liquides corporels, qui présente une ou plusieurs cassettes (2, 3, 4, 5) remplaçables, qui contiennent des fluides de fonctionnement, des agents de fonctionnement et/ou des agents consommables et peuvent être insérées dans des logements (17) appropriés de l'analyseur (1), l'analyseur (1) présentant un système (6) pour le renouvellement de l'air ambiant pour la gestion de la température de l'analyseur, lequel présente côté entrée de l'analyseur (1) une unité à filtre (7) pour la filtration de l'air ambiant à renouveler, **caractérisé en ce que** l'unité à filtre (7) est intégrée dans au moins l'une des cassettes (2, 3, 4, 5) remplaçables.

2. Analyseur (1) selon la revendication 1, **caractérisé en ce que** la cassette (3) remplaçable avec l'unité à filtre (7) intégrée présente un canal d'aération (8) qui est relié côté aspiration ou refoulement à un dispositif de transport d'air disposé dans l'analyseur (1), de préférence un ventilateur (11).

3. Analyseur (1) selon la revendication 2, **caractérisé en ce que** le canal d'aération (8) présente dans la cassette (3) amovible une ouverture de sortie (13) dirigée de préférence vers la zone de fond (22) de l'analyseur, ouverture dans la zone de laquelle est disposée l'unité à filtre (7), laquelle présente une surface d'afflux (23) dirigée horizontalement.

4. Analyseur (1) selon la revendication 3, **caractérisé en ce que** le canal d'aération (8) est guidé dans la cassette amovible (3) par exemple en forme de U et est en liaison côté entrée avec un canal d'aspiration (21), qui est disposé de préférence dans la zone de fond (22) de l'analyseur.

5. Analyseur (1) selon l'une quelconque des revendications 2 ou 4, **caractérisé en ce que** le canal de guidage d'air (8) est en liaison après l'insertion de la cassette (3) amovible dans l'analyseur avec une ouverture d'entrée (12) appropriée d'un canal de guidage d'air (24) dans l'analyseur (1).

6. Analyseur (1) selon la revendication 5, **caractérisé en ce que** le canal de guidage d'air (24) de l'analyseur est dirigé sur des composants (15, 16) à refroidir de l'analyseur (1).

7. Analyseur (1) selon l'une quelconque des revendications 1 à 6, **caractérisé en ce que** l'analyseur (1) présente au moins une cassette de détection (2) amovible, au moins un pack à fluide (3) amovible et au moins une cassette (4) remplaçable avec des fluides de contrôle de qualité, l'unité à filtre (7) étant intégrée de préférence dans le pack à fluide (3).

8. Analyseur (1) selon l'une quelconque des revendications 1 à 7, **caractérisé en ce que** l'unité à filtre (7) présente un filtre à couche de fibre, un filtre à lit tassé ou un filtre solide.

9. Cassette (2, 3, 4, 5), qui peut être insérée de façon amovible dans un analyseur (1), de préférence dans un analyseur pour l'analyse de liquides corporels, lequel analyseur présente un système (6) pour le renouvellement de l'air ambiant pour la gestion de la température de l'analyseur, et laquelle cassette contient des fluides de fonctionnement, des agents de fonctionnement et/ou agents consommables pour le fonctionnement de l'analyseur, **caractérisée en ce qu'**une unité à filtre (7) est intégrée dans la cassette (2, 3, 4, 5) amovible pour le filtrage de l'air ambiant à renouveler.

10. Cassette selon la revendication 9, **caractérisée en ce que** l'unité à filtre (7) présente un filtre à couche de fibre, un filtre à lit tassé et un filtre solide.

11. Cassette selon la revendication 9, **caractérisée en ce que** la cassette est un pack à fluide (3), qui contient des fluides fonctionnels utilisés pour le fonctionnement de l'analyseur, en particulier des solutions de calibrage, des solutions de lavage, des liquides de référence ou certaines solutions de réactifs utilisées pour le fonctionnement.

12. Cassette selon la revendication 9, **caractérisée en ce que** la cassette présente un canal de guidage d'air (8), qui peut être relié côté aspiration ou refoulement à un dispositif de transport d'air disposé dans l'analyseur (1), de préférence un ventilateur (11) et/ou des canaux de guidage d'air (21, 24) côté analyseur en liaison avec celui-ci.
